# EUROPÄISCHE PATENTSCHRIFT

(11) **EP 1 339 433 B1**
(45) Veröffentlichungstag und Bekanntmachung des Hinweises auf die Patenterteilung: **14.11.2007**
(21) Anmeldenummer: 01995575.6
(22) Anmeldetag: 07.12.2001
(51) Int. Cl.: A61K 48/00, C07K 14/47, A61P 35/00, A61P 35/04

(54) **EXPRESSION DER KERATINGENE 8 UND 18 ZUR THERAPIE VON TUMOREN, INSBESONDERE DES MAMMAKARZINOMS**
EXPRESSION OF KERATIN GENES 8 AND 18 FOR TREATING TUMOURS, IN PARTICULAR A MAMMARY CARCINOMA
EXPRESSION DES GENES DE KERATINE 8 ET 18 POUR LA THERAPIE DE TUMEURS, NOTAMMENT DU CARCINOME MAMMAIRE

(30) Priorität: 07.12.2000 DE 10061110
(43) Veröffentlichungstag der Anmeldung: 03.09.2003
(73) Patentinhaber: Schaller, Gerhard, 14197 Berlin (DE)
(72) Erfinder: Schaller, Gerhard, 14197 Berlin (DE)
(74) Vertreter: Baumbach, Friedrich
(86) Internationale Anmeldenummer: PCT/DE2001/004579
(87) Internationale Veröffentlichungsnummer: WO 2002/045755

(56) Entgegenhaltungen:
- BUEHLER H ET AL: "Overexpression of the keratin 18 gene results in reduced malignancy of human breast cancer cells." EUROPEAN JOURNAL OF CANCER, Bd. 35, Nr. SUPPL. 4, 1999, Seite S200 XP008007347 ECCO 10: The European Cancer Conference;Vienna, Austria; September 12-16, 1999 ISSN: 0959-8049
- PARK J W ET AL: "ANTI-HER2 IMMUNOLIPOSOMES FOR TARGETED THERAPY OF HUMAN TUMORS" CANCER LETTERS, NEW YORK, NY, US, Bd. 118, Nr. 2, 1997, Seiten 153-160, XP002929657 ISSN: 0304-3835 in der Anmeldung erwähnt
- BARIBAULT H ET AL: "Mid-gestational lethality in mice lacking keratin 8." GENES & DEVELOPMENT, Bd. 7, Nr. 7A, 1993, Seiten 1191-1202, XP008007380 ISSN: 0890-9369 in der Anmeldung erwähnt

## Beschreibung

Die Erfindung betrifft ein Mittel zur Therapie maligner Erkrankungen, insbesondere zur Verhinderung bzw. Hemmung der Metastasierung HER2-exprimierender solider Tumoren. Das erfindungsgemäße Mittel ist dadurch gekennzeichnet, dass es die Expression der Keratingene 8 und 18 (KERATIN 8, KERATIN 18) in Tumorzellen begünstigt. Bevorzugt handelt es sich um das KERATIN 18-Gen zur gentherapeutischen Behandlung, das insbesondere zur HER2-vermittelten in vivo Transfektion an eine mit Antikörpern gegen den HER2-Rezeptor bestückte Liposomenkapsel gebunden, vorliegt. Gegenstand der Erfindung ist somit die Verwendung des KERATIN 8- und KERATIN 18-Gens zur Herstellung eines Arzneimittels für die Therapie von soliden Tumoren, vorzugsweise des Mammakarzinoms. Anwendungsgebiete der Erfindung sind die Medizin und die pharmazeutische Industrie.

Brustkrebs ist die häufigste bösartige Erkrankung der Frau. Hierbei wird das Schicksal der Patientin durch die Metastasierung bestimmt. Ein erster Schritt bei der Ausbildung von Metastasen ist die Aufhebung der geweblichen Integrität und die Erhöhung der zellulären Verformbarkeit. Unmittelbare Substrate dieser funktionellen Veränderungen der Tumorzellen sind die Proteine der zellulären Adhäsionsstrukturen und des Zytoskeletts. Unter der Vielzahl der in diesem Zusammenhang beschriebenen Strukturen scheinen der E-Cadherin-Komplex der Adherens Junctions, die funktionellen Proteine der Desmosomen und die hier ankernden Keratine der Intermediärfilamente eine herausragende Rolle zu spielen.

Bei den Keratinen handelt es sich um eine Gruppe von bislang 20 bekannten Proteinen. Die Expression erfolgt sowohl gewebeals auch funktionsspezifisch (Franke 1981). In den Epithelzellen werden die Keratine in der Regel paarweise exprimiert, um als Heterodimere die Intermediärfilamente zu bilden, die wiederum Teil des Zytoskeletts sind. So läßt sich in den luminalen Zellen des zweireihigen Brustdrüsenepithels, entsprechend einer spezifischen Differenzierungsleistung, konstant das Keratinpaar 8 und 18 (KERATIN 8/18) nachweisen. Diese stringente paarweise Expression führt dazu, dass aus methodischen Gründen bei wissenschaftlichen Untersuchungen entweder das eine oder das andere Keratin untersucht wird (Baribault 1993). Vom Verhalten eines Keratins kann dann immer auf das Verhalten des anderen Partners geschlossen werden. Die Ausbildung eines funktionsfähigen Filaments ausschließlich aus KERATIN 8 oder KERATIN 18 ist nicht möglich.

Bei der Entstehung und -entwicklung v.a. solider Tumoren kommt es im Verlauf der malignen Transformation in der Regel zu einer Suppression der differenzierungsassoziierten Keratine. Ausgeglichen wird dieser Verlust durch eine vermehrte Expression anderer Keratine und von Vimentin. Der Austausch der Keratine 8/18 gegen das mesenchymale Intermediärfilamentprotein Vimentin geht einher mit einem Verlust an geweblicher Integrität, einer erhöhten Verformbarkeit der Zellen und daraus folgend einer zunehmenden Metastasierungsfähigkeit, wie *Vallés und Thiery* am Harnblasenkarzinom zeigen konnten (Vallés 1990). Wie wichtig die Expression von KERATIN 8/18 für die gewebliche Integrität ist, zeigen Experimente der Arbeitsgruppe von *Oshima:* Eine knockout-Mutation des KERATIN 8-Gens bei Mäusen führt zum intrauterinen Tod der Embryonen infolge von Blutungen aus mangelhaft strukturiertem Lebergewebe (Baribault 1993).
Aus diesen Untersuchungen ergibt sich, dass der Verlust von KERATIN 8/18 mit einer Verminderung geweblicher Integrität, aber auch mit einer erhöhten Verformbarkeit der betroffenen Zellen verbunden ist. Diese Änderungen der Zelleigenschaften erklären letztendlich auch die hohe Metastasierungsrate von Tumoren, die Zellen mit verminderter KERATIN 8/18-Expression aufweisen. Für das Mammakarzinom konnte ebenfalls nachgewiesen werden, dass die Expression von Vimentin in der Tumorzelle mit einer aggressiven Metastasierung verknüpft ist und damit zu einer schlechten Prognose führt (Thompson 1992, Sommers 1994).

Weiterhin wurde ein Zusammenhang zwischen der Überexpression des humanen KERATIN 18 und der Malignität von Brustkrebs beschrieben (Bühler 1999).

Die Neigung zur Metastasierung gilt in der Tumorklassifizierung als Merkmal der Malignität und ist u.a. eine Ursache dafür, dass viele Tumorerkrankungen trotz der enormen Fortschritte der Wissenschaft und Forschung in den letzten Jahren auf diesem Gebiet bis heute unheilbar sind. Die bisher etablierten Behandlungsmöglichkeiten, die auf eine chirurgische Entfernung des Tumorgewebes und eine Unterdrückung der zellteilung durch Chemotherapie oder radioaktive Bestrahlung abzielen, erscheinen nicht geeignet, die Heilungschancen maligner Tumorerkrankungen wesentlich zu verbessern.

Mehr Erfolg verspricht dabei die Gentherapie. Dieses relativ junge Therapiekonzept setzt bei den Ursachen an und versucht Defekte in Genen bzw. pathogene Regulationsmechanismen, die für die Tumorentstehung ursächlich sind, zu reparieren. Neben der Komplexität der Entstehung der meisten Tumoren, die ein gezieltes Reparieren defekter Gene erschweren, besteht bis heute die Schwierigkeit, ein effektives Einschleusen der Gene in die zu behandelnden Zellen sicherzustellen.

Rezeptoren, die sich vermehrt auf der Oberfläche der Tumorzellen befinden und diese somit eindeutig identifizieren, versprechen große Fortschritte bei der Verbesserung des Einschleusen therapierelevanter Gene. So wird HER2 (Human Epidermal Growth Factor Receptor) bei 25% aller Mammakarzinome überexprimiert. Während der Embryogenese ist HER2 bei der Entwicklung des Herzens und des Gehirns beteiligt. Im erwachsenen Organismus wird HER2 nur noch residual exprimiert. Patientinnen mit diesem Tumormerkmal haben eine besonders schlechte Prognose, da dieser Rezeptor aus der EGFR-Familie einerseits starke Proliferationssignale an den Zellkern vermittelt und andererseits durch Zerstörung der Zell-Zell-Kontakte des epithelialen Verbands zu besonders aggressiver Metastasierung führt.

Das therapeutische Ziel der bislang etablierten Therapien, wie Operation, Chemotherpie, Bestrahlung und Hormontherpie, aber auch der in der präklinischen Erprobung befindlichen Neoangiogenese- und Tyrosinkinasehemmer, ebenso wie das bereits zugelassene Trastuzumab (Herceptin™) ist die Hemmung der Proliferation.

Dabei bleibt außer acht, dass bei einer Vielzahl von Tumorerkrankungen vor allem die Folgen des Diffenzierungsverlustes in Form von Metastasenbildung für das häufige Auftreten von Rezidiven und somit für das Scheitern einer effektiven Therapie verantwortlich ist.

Ein wesentlicher Nachteil der auf die Proliferationshemmung abzielenden Therapien ist demzufolge, dass diese Therapieformen nicht geeignet sind, eine Verhinderung bzw. eine effektive Unterdrückung der Metastasierung von Tumoren zu gewährleisten.

Der Erfindung lag deshalb die Aufgabe zugrunde, ein Mittel zur gentherapeutischen Behandlung solider Tumoren zu finden und zur Anwendung bereitzustellen, das neben einer Proliferationshemmung durch eine Differenzierungsinduktion bzw. durch eine Verhinderung des Differenzierungsverlustes die Ausbildung von Metastasen effektiv unterdrückt.

Aus einer retrospektiven Studie an 134 duktalen Mammakarzinomen ist aufgrund einer immunhistochemischen Bestimmung bekannt, dass eine KERATIN 8/KERATIN 18-Expression im Tumor mit dem Überleben der Patientinnen korreliert (Leube 1986, Yamamoto 1990). Etwa jede fünfte Patientin war positiv (Score >4 aus maximal 12). Bis auf eine waren alle diese Patientinnen nach einem Beobachtungszeitraum von neun Jahren metastasenfrei. In einer parallel angelegten Studie an bisher 70 Endometriumkarzinom-Patientinnen fand sich eine ganz ähnliche Korrelation (Schaller 1996).

Diese Befunde konnten auch in vitro nachvollzogen werden. Untersucht wurde an mehreren etablierten Mammakarzinom-Zellinien die KERATIN 18-Expression, die Invasivität in der Boyden-Kammer und die Metastasierung in der athymischen Nacktmaus. Auch hier zeigte sich eine enge inverse Korrelation zwischen KERATIN 18-Expression und Malignität.

Die Erfindung basiert nun auf den eigenen Erkenntnissen, dass die Expression von KERATIN 18 in jedem Tumor, der sich aus einem zweireihigen Epithel entwickelt, ein ausgesprochenes Invasions- und Metastasierungshemmnis darstellt. Dies wiederum verdeutlicht, dass eine hohe KERATIN 18-Expression in einem Tumor mit einer signifikant besseren Prognose z.B. für die Brustkrebspatientin verknüpft ist, da in kultivierten Mammakarzinomzellen eine erhöhte KERATIN 18-Expression mit dramatisch reduzierter Malignität und Aggressivität der Zellen korreliert.

Die Aufgabe der Erfindung wird gemäß den Hauptansprüchen realisiert, die Unteransprüche sind vorzugsvarianten. Gegenstand der Erfindung ist insbesondere ein Mittel zur gentherapeutischen Behandlung von Tumoren, mit welchem man das KERATIN 8- und KERATIN 18-Gen im Tumor zur Überexpression bringt, das man nach an sich bekannten Methoden der Gentherapie in den Tumor einbringt und dort zur Überexpression bringt, wobei die Kombination beider Gene die Ausführungsform darstellt. Für die Gentherapie liegt die genetische Substanz zur direkten Anwendung bevorzugt in Formulierungen zur parenteralen Applikation oder besonders bevorzugt in Form von liposomalen Komplexen vor. In einer anderen Variante wird das Nukleotid in Form eines Vektors zur gentherapeutischen Anwendung bereitgestellt. Mit der vorliegenden Erfindung konnte erstmalig gezeigt werden, dass das KERATIN 8- und KERATIN 18-Gen und ihre entsprechenden Proteine differenzierungsassoziiert sind und damit der malignen Transformation diametral entgegenstehen.

In einer bevorzugten Ausführungsvariante der Erfindung wird das erfindungsgemäße Mittel, KERATIN 8- und KERATIN 18-Gen, in eine an sich bekannte HER2-Antikörper besetzte Liposomenkapsel als Carrier-Molekül eingebracht und kann mit diesem Carrier in die Tumorzelle überführt werden, wo es zur Überexpression gelangt. Zur Einschleusung von Chemotherapeutika bzw. Genkonstrukten in Tumorzellen ist die Verwendung dieser stark kationisch geladenen Liposomenkapsel, an die Antikörper gegen den HER2-Rezeptor gebunden sind, bereits beschrieben. Auch Teile eines Antikörpers, z.B. Fab-Fragmente des gentechnisch hergestellten und als Medikament seit dem 28.08.2000 in Europa zugelassenen humanisierten Antikörpers Trastuzumab (Herceptin™) sind zur Erkennung von Tumorzellen geeignet. Nachdem das HER2-Liposomen-Konstrukt an das Antigen gebunden hat, wird es durch die Tumorzelle internalisiert und der Inhalt der Liposomenkapsel freigesetzt (Kirpotin 1997, Park 1997). Seine Anwendung ist jedoch bisher auf die Hemmung der Proliferation beschränkt.

Die erfindungsgemäße Gentherapie mit dem KERATIN 8 und KERATIN 18 -Gen ermöglicht überraschend eine Kombination aus zuverlässiger Proliferationshemmung, der sich erfindungsgemäß eine Induktion von Differenzierung anschließt, wodurch eine zuverlässige und nachhaltige Behandlung aller solider Tumoren, die sich aus einem zweireihigen Epithel entwickeln, und v.a. des Mammakarzinoms möglich ist.

So gelingt es gemäß der Erfindung bereits in der Tumorzelle in die G0-Phase des Zellzyklus einzugreifen und dadurch die Differenzierung zu induzieren bzw. einen Verlust der Differenzierung zu verhindern, wodurch die Ausbildung von Metastasen effektiv unterdrückt werden kann. Abhängig vom Grad der HER2-Expression im Tumor und einer Nachbarschaftsinduktion läßt sich eine drastische (> 90%) Reduktion der Malignität erzielen.

Vorteile des erfindungsgemäßen Mittels zur Gentherapie durch HER2-vermittelte KERATIN 8/KERATIN 18-Transfektion sind:
1. im Gegensatz zur Proliferationshemmung werden auch Tumorzellen in der GO-Phase mit der HER2-vermittelten Gentherapie erreicht.
2. Diese Therapie ist prinzipiell für alle soliden Tumoren anwendbar, die KERATIN 8 und KERATIN 18 im Ausgangsepithel und HER2 im Tumor exprimieren. Dies trifft für alle Tumoren, die sich aus einem zweireihigen Epithel entwickeln, zu.
3. Die Induktion von Differenzierung entspricht der physiologischen Entwicklung und gleicht den malignen Differenzierungsverlust aus.

### Literatur:

Baribault H. et al., Mid-gestational lethality in mice lacking keratin 8 Genes & Development 7 (1993) 1191
Bühler H. et al., Overexpression of the keratin 18 gene results in reduced malignancy of human breast cancer cells European Journal of Cancer, Bd. 35, Nr. Suppl. 4, 1999, S. 200
Kirpotin D. et al., Streically stabilized anti-HER2 immunoliposomes: Design and targeting to human breast cancer cells in vitro Biochemistry 36 (1997) 66
Leube RE. et al., Cytokeratin expression in simle epithelia III. Detection of mRNAs encoding human cytokeratins nos. 8 and 18 in normal and tumor cells by hybridisation with cDNA sequences in vitro and in situ Differentiation 33 (1986) 69
Moll R. et al., The Catalog of human cytokeratins: patterns of expression in normal epithelia, tumors and cultured cells Cell 31 (1982) 11
Nakamura Y. , Cleaning up on β-catenin Nature medicine 3 (1997) 499
Park J. W. et al., Anti-HER2 immunoliposomes for targeted therapy of human tumors Cancer Letters 118 (1997) 153
Thompsom E. W. et al., Association of increased basement membrane invasiveness with absence of estrogen receptor and expression of vimentin in human breast cancer cell lines J Cellular Physiology 150 (1992) 534
Schaller G. et al., Elevated Keratin 18 protein expression indicates a favorable prognosis in patients with breast cancer Clin Cancer Res 2 (1996) 1879
Slamon D. J. et al., Human Breast Cancer: Correlation of Relapse and Survival with Amplification of the HER-2/neu Oncogene Science 235 (1987) 177
Vallés A. M. et al., Acidic fibroblast growth factor is a modulator of epithelial plasticity in rat bladder carcinoma cell line Proc Natl Acad Sci USA 87 (1990) 1124
Yamamoto R. et al. Cloning and Swquence of cDNA for Human Placental Cytokeratin 8. Regulation of the mRNA in Trophoblastic Cells by cAMP
Anschließend wird die Erfindung durch 2 Beispiele für Keratin 18 näher erläutert.

### Beispiel 1

Durch Transfektion des KERATIN 18-Gens in die Mammakarzinom-Zellinie MDA-MB 231 wurde eine Erhöhung der KERATIN 18-Expression erzielt, wodurch eine verringerte Malignität der Tumorzellen erreicht wurde, die gekennzeichnet ist:
a) durch eine gesteigerte zelluläre Adhäsivität,
b) induzierte Adhäsionsproteine,
c) geringere Proliferation im Softagar,
d) geringere Invasivität in der Boydenkammer,
e) kleinere Tumoren in einer Nacktmaus.

### Beispiel 2

Weitere Untersuchungen mit einem KERATIN 18-positiven Subklon der normalerweise KERATIN 18-negativen jedoch mit der KERATIN 18-Gen transfizierten Mammakarzinom-Zellinie ergaben ebenfalls eine drastisch (>90%) reduzierte Malignität des KERATIN 18-positiven Klons in Form einer erhöhten Expression von desmosomalen Proteinen, einer fehlenden Invasivität in der Boydenkammer und einer fehlenden Metastasierung in der Nacktmaus.

## Patentansprüche

1. Mittel zur gentherapeutischen Behandlung von HER2-exprimierenden soliden Tumoren unter gleichzeitiger Hemmung und Verhinderung der Tumormetastasierung, **dadurch gekennzeichnet, dass** es sich aus dem KERATIN 8- und KERATIN 18-Gen zusammensetzt, die in den Tumorzellen transfiziert werden, vorzugsweise in der GO-Phase des Zellzyklus.

2. Mittel gemäß Anspruch 1, **dadurch gekennzeichnet, dass** es zur Transfektion in Tumorzellen geeignet ist, die auf der Zelloberfläche den HER2-Rezeptor aufweisen.

3. Mittel gemäß Anspruch 1, **dadurch gekennzeichnet, dass** die genetische Substanz zur Anwendung in vivo vorliegt, vorzugsweise in Formulierungen zur parenteralen Applikation oder in Form von liposomalen Komplexen.

4. Mittel gemäß Anspruch 1, **dadurch gekennzeichnet, dass** das Nukleotid in Form eines Vektors zur gentherapeutischen Anwendung vorliegt.

5. Mittel gemäß Anspruch 3, **dadurch gekennzeichnet, dass** der liposomale Komplex eine mit Antikörpern gegen den HER2-Rezeptor bestückte Liposomenkapsel, bevorzugt eine stark kationisch geladene Liposomenkapsel, ist.

6. Mittel gemäß Anspruch 5, **dadurch gekennzeichnet, dass** als Carrier Trastuzumab verwendet wird.

7. Verwendung des Mittels _gemäß den Ansprüchen 1 bis 6 zur Herstellung eines Arzneimittels für die Gentherapie von soliden Tumoren, die sich aus einem zweireihigen Epithel entwickeln.

8. Verwendung gemäß Anspruch 7 zur Gentherapie von HER2-exprimierenden soliden Tumoren, bevorzugt von Mammakarzinomen.

9. Verfahren zur Herstellung von einem pharmazeutischen Mittel gemäß einem der Ansprüche 1 bis 6, **dadurch gekennzeichnet, dass** man die therapeutische Menge mindestens eines Nukleotids mit an sich bekannten Hilfs- und Trägerstoffen nach an sich üblichen technischen Herstellungsverfahren kombiniert und in die gewünschte Formulierung überführt.

## Claims

1. Agent for the therapeutic treatment of tumours by simultaneously inhibiting and preventing tumour metastases, **characterised by** the fact that it increases the expression of the K8 and/or K18 gene, which are transfected in the tumour cells, preferably in the G0 phase of the cell cycle.

2. Agent according to claim 1, **characterized by** the fact that it is suitable for transfection in tumour cells, which display the HER2 receptor on the cell surface.

3. Agent according to claim 1, **characterized by** the fact that the genetic substance is to be found in vivo, preferably in formulations for parenteral application or in the form of liposomal complexes.

4. Agent according to claim 1, **characterized by** the fact that the nucleotide is present in the form of a vector for gene therapeutic application.

5. Agent according to claim 3, **characterized by** the fact that the liposomal complex is a capsule of liposomes equipped with antibodies against the HER2 receptor, preferably a capsule of liposomes strongly cationic-loaded.

6. Agent according to claim 5, **characterized by** the fact that Trastuzumab is used as a carrier.

7. The use of agent according to the claims 1 to 6 for the production of a pharmaceutical agent for the gene therapy of solid tumours, which have developed from a two row epithelia.

8. The use according to claim 7 for the gene therapy of solid tumours that express HER2, preferably of breast cancer.

9. Procedures for the production of a pharmaceutical agent agent according to one of the claims 1 to 6, **characterized by** the fact that the therapeutic quantity of at least one nucleotide is combined with currently known auxiliary and carrier substances, manufactured by the current and usual production techniques, and this combination is then transferred into the desired formulation.

## Revendications

1. Produit destiné au traitement génique de tumeurs solides exprimant HER2, sous inhibition et prévention simultanées de migration métastatique, **se caractérisant par le fait que** ce produit se compose du gène de la KÉRATINE 8 et du gène de la KÉRATINE 18 qui sont transférés dans les cellules tumorales, de préférence dans la phase G0 du cycle cellulaire.

2. Produit selon la revendication 1, **se caractérisant par le fait qu'**il est approprié à la transfection dans des cellules tumorales qui présentent à la surface de la cellule le récepteur HER2.

3. Produit selon la revendication 1, **se caractérisant par le fait que** la substance génétique pour l'application *in vivo* existe, de préférence dans des formulations pour une application parentérale ou sous forme de complexes liposomaux.

4. Produit selon la revendication 1, **se caractérisant par le fait que** le nucléotide existe sous forme d'un vecteur pour l'application en thérapie génique.

5. Produit selon la revendication 3, **se caractérisant par le fait que** le complexe liposomal est une capsule de liposome munie d'anticorps contre le récepteur HER2, de préférence une capsule de liposome fortement chargée en cations.

6. Produit selon la revendication 5, **se caractérisant par le fait que** le Trastuzumab est utilisé en tant que porteur.

7. Emploi du produit selon les revendications 1 à 6 pour la fabrication d'un médicament pour la thérapie génique de tumeurs solides qui se développent à partir d'un épithélium en deux couches.

8. Emploi selon la revendication 7 pour la thérapie génique de tumeurs solides exprimant HER2, de préférence de cancers du sein.

9. Procédé de fabrication d'un produit pharmaceutique selon l'une des revendications 1 à 6, **se caractérisant par le fait que** l'on combine la quantité thérapeutique d'au moins un nucléotide avec des agents auxiliaires et substances porteuses connues selon un procédé de fabrication technique usuel en soi et que l'on transforme ceci dans la formulation souhaitée.
